# EUROPEAN PATENT APPLICATION

(11) **EP 1 849 491 A1**
(43) Date of publication of application: **31.10.2007**
(21) Application number: 07251740.2
(22) Date of filing: 25.04.2007
(51) Int. Cl.: A61M 16/06

(54) **Oral nasal cannula**

(30) Priority: 25.04.2006 US 410503
(71) Applicant: Oridion Medical 1987 Ltd., Jerusalem 91450 (IL)
(72) Inventor: Porat, Ron, D.N. Haela 99875 (IL)
(74) Representative: Hedley, Nicholas James Matthew

(57) **Abstract**

There is provided a cannula (10) which may include a spacer (25) adapted to maintain a minimum distance between at least a portion of an oral cavity and at least a portion of an oral breath collector (22) and at least one nasal breath collector (18). There is also provided a method for sampling exhaled breath including positioning a cannula on a face of a subject, wherein the cannula may include a spacer adapted to maintain a minimum distance between at least a portion of an oral cavity and at least a portion of an oral breath collector and at least one nasal breath collector.

## Description

### BACKGROUND

Different types of oral/nasal cannulas are generally used to deliver oxygen to patients who require assistance to breath properly, to collect carbon dioxide samples from patients to monitor respiration, or to perform both functions. Such cannulas are used when direct ventilation is not provided. The term "oral/nasal" refers to the adaptable configuration of such cannulas which are designed to be in close proximity to the oral cavity and/or nasal cavity and may also be at least partially inserted into the nasal cavity.

In either arrangement, cannulas are designed so that a sidestream of a patient's exhaled breath may flow through the cannula to a gas analyzer to be analyzed. The results of this analysis may provide an indication of the patient's condition, such as the state of the patient's pulmonary perfusion, respiratory system and metabolism. An example of a gas analysis often performed is capnography using an analyzer called a capnograph. Capnography is the monitoring of the time dependent respiratory carbon dioxide (CO₂ concentration, which may be used to directly monitor the inhaled and exhaled concentration of CO₂ and indirectly monitor the CO₂ concentration in a patient's blood. Capnography may provide information about CO₂ production, pulmonary (lung) perfusion, alveolar ventilation (alveoli are hollow cavities in the lungs in which gas exchange is being performed) and respiratory patterns. Capnography may also provide information related to a patient's condition during anaesthesia, for example by monitoring the elimination of CO₂ from anaesthesia breathing circuit and ventilator.

More information regarding capnography may be found in hftp://www.capnography.com/, which is herein incorporated by reference in its entirety.

In oral breath measurements, including capnography, the location of the oral breath collector is crucial, however the location may change during one sampling process or between one sampling process to another. The oral breath collector may also be aspirated or partially aspirated into the subject's mouth and thus may cause undesired effects such as, hindering the flow of exhaled and/or inhaled breath, interfering with the breath sampling and in turn causing inaccurate breath test results and discomfort to the subject being examined. Moreover, it is extremely desirable to have repeatability sampling (and/or testing) periods. One repeatability issue occurs longer breath tests of a single subject, where the position of the cannula, for example, the position of the oral collector collecting point(s) (such as the end of one or more tube(s)) will be maintained in the same spacial position relative to the exhaled oral breath stream. A second repeatability issue is repeatability over different testing periods for the same subject, as testing occurring during different days, where the same spatial position noted above should be essentially maintained to provide a more optimum comparison of results for the subject during different sampling processes (and/or tests).

The use in the art of various disposable devices, such as cannulas of Salter Labs, Arvin, Calif. USA, and Novametrix Medical Systems, Inc., which use a collection tube which is "cut to fit" for each patient at the time of use, and usually involve spacial positioning by means of partially deformable wire, do not well satisfy the needs for repeatable positioning of the collection point(s) within a subject's oral breath system. Where deferring attendants (technicians, nurses and the like) will attempt to measure and cut a breath collection tube to a length that places the end in the relative center of an oral breath stream, each test will be subject to variations in length of the tube cut by different attendants. Even with the same attendant, time dilated tests (such as performed by the same attendant on the same patient over different day) will invariably result in new "disposable" oral breath collectors being cut to somewhat different length (height). Further exacerbating the position variation (non-repeatability) issue, is that the front/back and side/side positioning by hand manipulation of the wire-supporting the tube is not mechanically repeatable for position within the expected oral breath stream. Further, the (semi) flexible wire supporting the tube may be deflected during the sampling procedure. Even with the use of a collection funnel to better control the focusing of exhaled breath, the spacial position (height, front/back and side/side) within the funnel is important.

There is thus a widely recognized need for, and it would be highly advantageous to have, a cannula in which the location of the oral breath collector is maintained during the sampling procedure and/or which may be repeatable during or between sampling and/or which maintain a minimum distance between the oral cavity and the oral breath collector.

The foregoing examples of the related art and limitations related therewith are intended to be illustrative and not exclusive. Other limitations of the related art will become apparent to those of skill in the art upon a reading of the specification and a study of the figures.

### SUMMARY

The following embodiments and aspects thereof are described and illustrated in conjunction with systems, tools and methods which are meant to be exemplary and illustrative, not limiting in scope. In various embodiments, one or more of the above-described problems have been reduced or eliminated, while other embodiments are directed to other advantages or improvements.

According to some embodiments, there is provided a cannula which may include a spacer adapted to maintain a minimum distance between at least a portion of an oral cavity and at least a portion of an oral breath collector. The cannula may further include at least one nasal breath collector.

There is also provided according to some embodiments, a cannula which may include a spacer adapted to maintain a minimum distance between at least a portion of an oral cavity and at least a portion of an oral breath collector, wherein the cannula may be adapted to be coupled to a breath test analyzer. The cannula may further include at least one nasal breath collector. The cannula may be connected to a breath test analyzer by one or more collection tube(s).

There is also provided according to some embodiments, a method for sampling exhaled breath including positioning a cannula on a face of a subject, wherein the cannula may include a spacer adapted to maintain a minimum distance between at least a portion of an oral cavity and at least a portion of an oral breath collector. The cannula may further include at least one nasal breath collector.

In accordance with a further preferred embodiment of the present disclosure the inner surface includes a plurality of flow surfaces each having a different flow direction.

### BRIEF DESCRIPTION OF THE FIGURES

Exemplary embodiments are illustrated in referenced figures. It is intended that the embodiments and figures disclosed herein are to be considered illustrative, rather than restrictive. The disclosure, however, both as to organization and method of operation, together with objects, features, and advantages thereof, may best be understood by reference to the following detailed description when read with the accompanying figures, in which:
Fig. 1A is a simplified front-view pictorial illustration of an oral nasal sampling cannula constructed and operative in accordance with a preferred embodiment of the present disclosure; Figs. 1B and 1C are simplified rear-views pictorial illustrations of an oral nasal sampling cannula constructed and operative in accordance with a preferred embodiment of the present disclosure;
Figs. 2A and 2B and 2C are partial perspective illustrations with simplified sectional illustrations taken along section lines: IIA-IIA (in Fig. 2B, the line IIA-IIA is taken on the whole part), IIB-IIB (in Fig. 1A) and IIC-IIC (in Fig. 2B, the line IIC-IIC is taken on the whole part);
Figs. 3A, 3B and 3C are schematic illustrations of gas flow in the oral nasal sampling cannula of Figs. 1A - 2C, wherein Fig. 3A depicts oxygen flow and Figs. 3B and 3C depict sampling of exhaled breath;
**Figs. 4A and 4B** are simplified front-view and rear-view pictorial illustrations of an oral nasal sampling cannula having a single nasal prong, constructed and operative in accordance with another preferred embodiment of the present disclosure;
**Figs. 5A, 6B and 5C** are partial perspective illustrations with simplified sectional illustrations taken along section lines: VA-VA (in Fig. 5B, the line VA-VA is taken on the whole part), VB-VB (in Fig. 4A), and VC-VC (in Fig. 5B, the line VC-VC is taken on the whole part);
**Figs. 6A, 6B and 6C** are schematic illustrations of gas flow in the oral nasal sampling cannula of Figs. 4A - 5C, wherein Fig. 6A depicts oxygen flow and Figs. 6B and 6C depict sampling of exhaled breath;
**Figs. 7A and 7B** are simplified front-view and rear-view pictorial illustrations of an oral nasal sampling cannula having an enlarged oral scoop, constructed and operative in accordance with yet another preferred embodiment of the present disclosure;
**Figs. 8A, 8B and 8C** are partial perspective illustrations with simplified sectional illustrations taken along section lines: VIIIA-VIIIA (in Fig. 8B, the line VIIIA-VIIIA is taken on the whole part), VIIIB-VIIIB (in Fig. 7A) and VIIIC-VIIIC (in Fig. 8B, the line VIIIC-VIIIC is taken on the whole part); and
**Figs. 9A, 9B and 9C** are schematic illustrations of gas flow in the oral nasal sampling cannula of Figs. 7A - 8C, wherein Fig. 9A depicts oxygen flow and Figs. 9B and 9C depict sampling of exhaled breath.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated within the figures to indicate like elements.

### DETAILED DESCRIPTION

While a number of exemplary aspects and embodiments have been discussed above, those of skill in the art will recognize certain modifications, permutations, additions and sub-combinations thereof. It is therefore intended that the following appended claims and claims hereafter introduced be interpreted to include all such modifications, permutations, additions and sub-combinations as are within their true spirit and scope.

The present disclosure relates to a nasal cannula and to an oral/nasal cannula, and, more particularly, to a nasal cannula and an oral/nasal cannula which permits both delivery of oxygen and accurate sampling of carbon dioxide. The present disclosure seeks to provide an improved cannula for use in gas sampling, such as with a capnographic system.

For purposes of description, the discussion herein is focused on cannulas for use with human patients, it being understood that the present disclosure is not limited in scope only to use with patients and can beneficially be used in various other contexts.

In one embodiment, there is provides a cannula which may include a spacer adapted to maintain a minimum distance between at least a portion of an oral cavity and at least a portion of an oral breath collector. The cannula may further include at least one nasal breath collector.

The term "oral cavity" may refer to any part of the mouth and/or the lips.

The spacer may be adapted to maintain a minimum distance between at least a portion of an oral cavity, for example, the upper lip of a subject and/or the facial part between the lower part of the nostril(s) and the upper lip and at least a portion of an oral breath collector. During breath sampling using existing cannulas positioned on a subject's face, the oral breath collector may also be aspirated or partially aspirated into the subject's mouth and thus may cause undesired effects such as, hindering the flow of exhaled and/or inhaled gases, interfering with the breath sampling and in turn causing inaccurate breath test results and also causing unpleasant feeling to the subject being examined. The cannulas provided herein are structured with a spacer that may be adapted to prevent or reduce this problem by at least partially separating between the subject's oral cavity and at least a portion of an oral breath collector. Conducting a breath test analysis using the cannulas provided herein may thus provide a more accurate and a more convenient breath analysis.

A "spacer", as referred to herein may include any item that may provide a minimum distance between two objects. The spacer may be shaped in the form of one or more wedge(s), bar(s), pin(s), column(s), cantilevered beam(s), nib(s) and/or other mechanical spacing structure(s). The wedge may include two edges, an upper edge and a lower edge, wherein the "upper edge" is the edge closer to the nostrils and the "lower edge" is the edge closer to the oral cavity. The upper edge may be thinner than the lower edge. This structure may allow securing the cannula to the subject's face, specifically in proximity to the nostrils, and allowing a minimum distance between the oral breath collector and the oral cavity and thus prevent or inhibit the penetration of the oral breath collector into the subject's mouth.

The spacer may be integrally formed with at least a part of the cannula. The spacer may be connected to the cannula by one or more points and may also be cantilevered. The spacer may be attached to the cannula. The spacer may be structured as an add-on to the cannula. The spacer may include one or more support elements and/or connecting elements that may be adapted to connect the spacer to the cannula. The spacer(s) may be formed of one or more material(s) including silicon, rubber, plastic, other polymeric material, metal, glass or any other appropriate material(s). The spacer(s) may include flexible, rigid, plastic, elastic parts or a combination thereof. The spacer may be adaptable and/or able to modify to fit to a certain feature in a subject's face, such as the upper lip and/or the facial part between the lower part of the nostril(s) and the upper lip.

The oral breath collector may include a scoop adapted to collect orally exhaled breath. The oral breath collector may include a prong adapted to collect orally exhaled breath. The oral breath collector may be adapted to direct oral exhaled toward a suction port. The oral breath collector may include an inner surface which may be configured to direct breath, exhaled from the mouth of a subject in substantially any direction, toward a suction port. The inner surface may include a plurality of flow surfaces each having a different flow direction. The oral breath collector may be adapted to cover a substantial portion of the mouth of a subject.

The breath exhaled from a subject being examined may be orally collected using an oral breath collector, nasally collected using a nasal breath collector or both. The nasal breath collector may include a nasal prong, which may be adapted to be at least partially inserted into a nostril.

The cannula may also include at least one oral oxygen delivery port. The at least one oral oxygen delivery port may include a plurality of oxygen delivery holes formed in said main body portion. The at least one oral oxygen delivery port may be adapted to deliver oxygen around the oral breath collector (for example, the scoop). The at least one oral oxygen delivery port may formed over the oral breath collector (for example, the scoop).

The cannula may further include at least one nasal oxygen delivery port. The at least one nasal oxygen delivery port may include a plurality of oxygen delivery holes. The at least one nasal oxygen delivery port may include a nasal oxygen delivery prong adapted to be at least partially inserted into a nostril of a subject. The nasal oxygen delivery prong may be shorter than the at least one nasal prong (the at least one nasal breath collector) and may be adapted to be at least partially inserted into a nostril of a subject.

The cannula may further include a separator, adapted to maintain a predetermined distance between the at least one nasal oxygen delivery port and a nostril of a subject.

The cannula may be structured with an angle between at least one oral breath collector and the nasal breath collector (this angle is also referred to herein as angle α). The angle may be in the range of 100 - 170 degrees, for example, in the range of 115 - 145 degrees, in the range of 125 - 145 degrees, in the range of 125 - 135 degrees, in the range of 130 - 140 degrees or about 135 degrees. The cannula structured with a certain angle α, as described herein, may be made comfortable to the subject undergoing examination when placed on the face.

The cannula may be structured with an angle between the axis of revolution of the interior part of the oral breath collection bore and the axis of revolution of the interior part of at least one nasal breath collector prong (this angle is also referred to herein as angle β). The angle may be in the range of 100 - 170 degrees, for example, in the range of 115 - 145 degrees, in the range of 125 - 145 degrees, in the range of 125 - 135 degrees; in the range of 130 - 140 degrees or about 135 degrees. The cannula structured with a certain angle β, as described herein, may allow a desirable flow of the fluid being sampled.

It is noted that one measure for repeatability between multiple sampling for a single subject and/or sampling (for example for statistical purposes) between various subjects may be achieved by standardizing one ore more of the features of: a) the distance between the base of the nose to the point(s) of oral breath collection ("height"); b) the distance from the oral breath production (such as the lips) to the point(s) of oral breath collection (''front/back'').

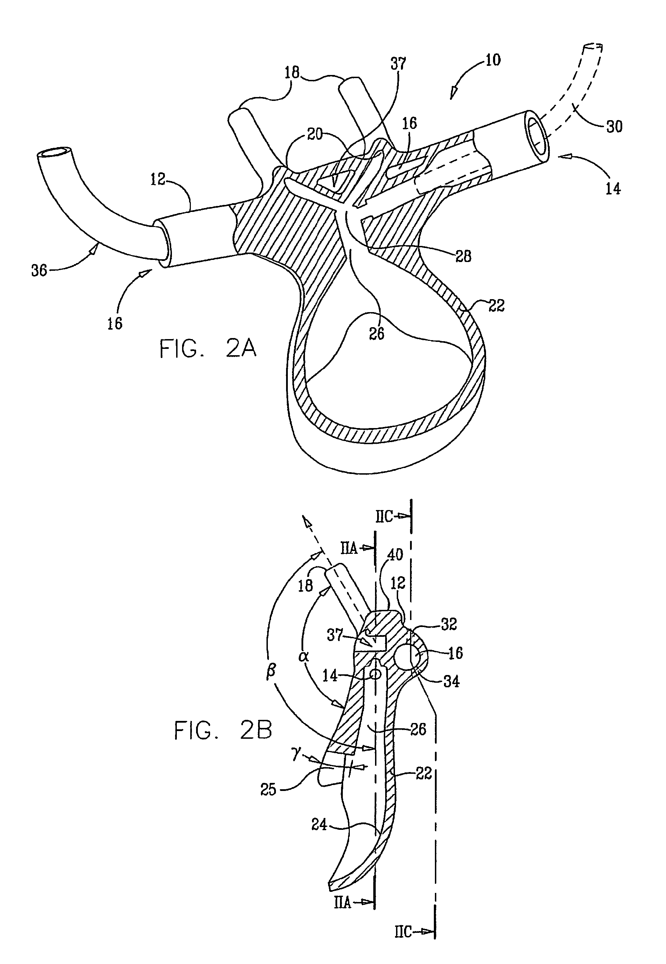

In order to better maintain repeatability of height and front/back position noted above a derivative or related function such as an angle on a nominally triangle or wedge shaped spacing device (spacer) may be used. As an example, the cannula may be structured with an angle of formation of the spacer (wedge), shown as angle γ (as depicted for example in Figs 2B, 5B and 8B) in order to better maintain the location of the oral breath collector, provide repeatability during or between sampling and/or maintain a minimum distance between the oral cavity and the oral breath collector. By forming a wedge of a generally angular configuration (such as but not limited to a wedge) the angle γ forms a pre-selected and repeatable spacing for a subject as the lips and nose position will not substantially change from test to test.

The angle γ may be in the range of 1 - 45 degrees, for example, in the range of 1 - 20 degrees, in the range of 5-10 degrees, in the range of 2-8 degrees or about 8 degrees.

The cannulas referred to herein may be used for sampling the breath of subject(s), especially for the purpose of providing capnographic data concerning the subject.

There is also provided in accordance with some embodiments, an oral nasal cannula for sampling breath of a subject, including a main body portion, having formed therein a suction port which is adapted to be connected to a suction device for side sampling of exhaled breath of the subject, at least one nasal prong integrally formed with the main body portion and adapted to collect nasally exhaled breath of the subject and an oral scoop, integrally formed with the main body portion and adapted to collect orally exhaled breath of the subject.

The main body portion may be formed with at least one oral oxygen delivery port and at least one nasal oxygen delivery port. Preferably, the at least one nasal oxygen delivery port includes a plurality of oxygen delivery holes formed in the main body portion. Alternatively, the at least one nasal oxygen delivery port includes at least one oxygen delivery prong which is integrally formed with the main body portion, which is shorter than the at least one nasal prong and is adapted to be at least partially inserted into a nostril of the subject.

The cannula(s) may also include a separator, adapted to distance the at least one nasal oxygen delivery port from the nose of the subject when the oral nasal cannula is placed on the face of the subject. The at least one oral oxygen delivery port may be formed over the oral scoop. The at least one oral oxygen delivery port may be directed sideways, such that delivered oxygen is directed around the oral scoop.

The cannula may include a main body portion, having formed therein a suction port which may be adapted to be connected to a suction device for side sampling of exhaled breath of a subject, at least one nasal prong integrally formed with the main body portion and adapted to collect nasally exhaled breath of the subject and an oral scoop, integrally formed with the main body portion and adapted to collect orally exhaled breath of a subject, wherein the cannula includes a spacer adapted to maintain a minimum distance between at least a portion of an oral cavity and at least a portion of the oral scoop.

There is also provided according to some embodiments, a cannula which may include a spacer adapted to maintain a minimum distance between at least a portion of an oral cavity and at least a portion of an oral breath collector, wherein the cannula may be adapted to be coupled to a breath test analyzer. The cannula may further include at least one nasal breath collector. The cannula may be connected to a breath test analyzer by one or more collection tube(s).

There is also provided according to some embodiments, a method for sampling exhaled breath including positioning a cannula on a face of a subject, wherein the cannula may include a spacer adapted to maintain a minimum distance between at least a portion of an oral cavity and at least a portion of an oral breath collector. The cannula may further include at least one nasal breath collector.

The method may further include at least partially inserting said at least one nasal breath collector into a nostril. The method may further include sampling exhaled breath collected from said oral cavity, said nostril or both using a gas analyzer coupled to said cannula. The method may further include providing oxygen through a nasal oxygen delivery port, an oral oxygen delivery port or both.

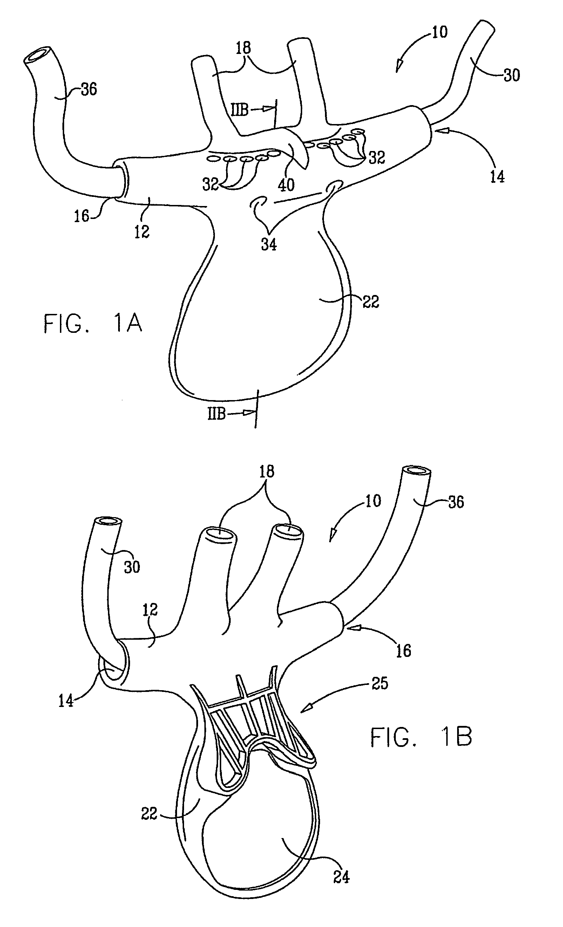

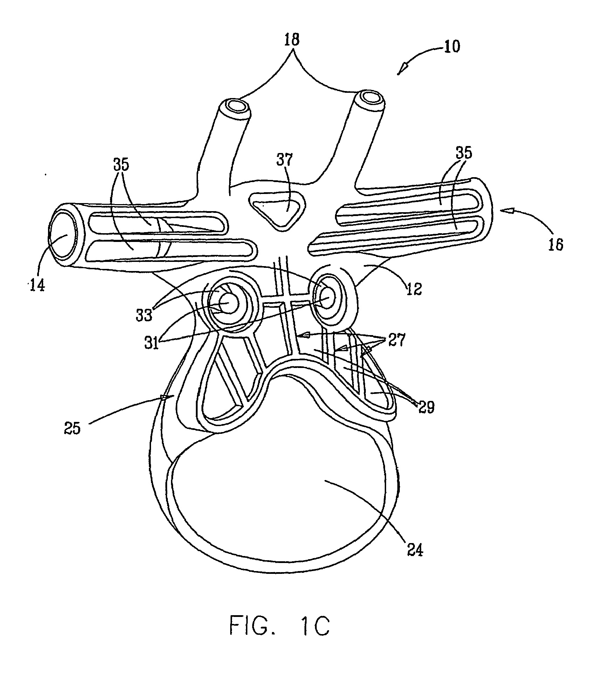

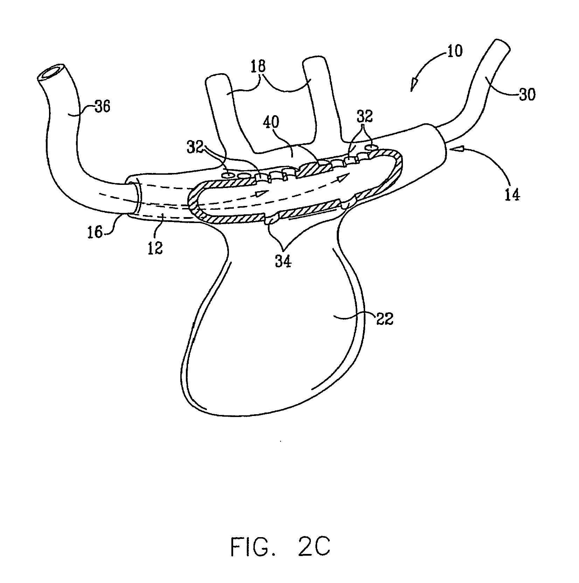

Reference is now made to Figs. 1A-2C. Fig. 1A is a simplified front-view pictorial illustration of an oral nasal sampling cannula constructed and operative in accordance with a preferred embodiment of the present disclosure. Figs. 1B and 1C are simplified rear-views pictorial illustrations of an oral nasal sampling cannula constructed and operative in accordance with a preferred embodiment of the present disclosure. Figs. 2A, 2B and 2C are simplified sectional illustrations taken along section lines: IIA-IIA (in Fig. 2B), IIB-IIB (in Fig. 1A) and IIC-IIC (in Fig. 2B). Figs. 1A - 2C show an oral nasal sampling cannula 10, which is adapted for collection of gases, such as carbon dioxide, exhaled by a subject, and for supplying oxygen to the subject. The oral nasal sampling cannula 10 is adapted to sample orally, nasally exhaled breath, or both.

The oral nasal sampling cannula 10 comprises a main body portion 12, having formed therein an exhaled breath collection bore 14 and an oxygen delivery bore 16. A pair of hollow nasal prongs 18, having inner ends 20 which are in fluid flow communication with a pair of nasal breath collection bores 21, is adapted for at least partial insertion into the nostrils of the subject and may be integrally formed with the main body portion 12.

An oral scoop element 22, including an internal surface 24, a spacer, formed in the shape of a wedge 25 adapted to maintain a minimum distance between a portion of an oral cavity and a portion of the oral scoop 22. The surface of the wedge 25 may be non-smooth, contoured and/or include structural elements such as rigids, holes, bars, nibs and the like, to form additional structural rigidity, to allow fixed seating against the face (for example, the lip), to allow moisture (for example, sweat) evaporation, to allow fixed seating against the face for subjects having facial hair, to provide comfort and/or to avoid sliding (for example, lateral sliding) of the oral nasal sampling cannula 10 on the face of the subject being examined. Fig. 1B shows examples of rigids 27 that may form spaces 29 between them. Fig. 1C shows examples of rigids 27 that may form spaces 29 between them, holes 33 that may have pins 31 extending all the way or part of the way through them, hole 37 (also shown in 2A and 2B), spaces 35 or any other element. The oral scoop element 22 may be integrally formed with main body portion 92. The oral scoop element 22 terminates at a top portion thereof in an oral breath collection bore 26 (Figs. 2A and 2B), which is in fluid flow connection with nasal breath collection bores 21, thereby forming an essentially single junction 28 (Fig. 2A). The junction can also be located closer to one prong 18 than to the other. The junction can also be located above the position shown in Fig. 2A or in any other place that would allow the desired fluid flow. Fig. 2C shows the space 15 extending from the oxygen delivery bore 16, which is in fluid flow communication with an oxygen delivery tube 36, and exits the oral nasal sampling cannula at nasal and oral oxygen delivery openings 32 and 34, toward the nose and mouth of the subject.

Single junction 28 is in fluid flow communication with exhaled breath collection bore 14, which in turn is in fluid flow communication with-an exhaled breath collection tube 30, which is adapted to be connected to a suctioning pump, such as that used in a side-stream capnograph (not shown), for example Microcap®, which is commercially available from Oridion BreathlD of Jerusalem, Israel.

Main body portion 12 includes, preferably at a forward facing surface thereof or alternatively at any other suitable location, nasal oxygen delivery openings 32 and may optionally also include oral oxygen delivery openings 34, both nasal and oral oxygen delivery openings being in fluid flow communication with oxygen delivery bore 16, as seen with particular clarity in Fig. 2B. Oxygen delivery bore 16 is in fluid flow communication with an oxygen delivery tube 36, which is adapted to be connected to a source of oxygen (not shown).

The hatch lines may refer to one or more material(s) including silicon, rubber, plastic, other polymeric material, metal, glass or any other material(s).

Oxygen delivery tube 36 and exhaled breath collection tube 30 may optionally be placed around the ears of the subject, thereby stabilizing the oral nasal sampling cannula 10 on the subject's face.

As seen clearly in Fig, 1A, a separator 40 is integrally formed with main body portion 12 at a forward facing surface thereof, Separator 40 is adapted to engage the nose of the subject, thereby distancing the nose from nasal oxygen delivery openings 32 and ensuring that a sufficient oxygen supply reaches the subject's nose, while not closing off the subject's nasal opening, which would incur a resistance to air flow during exhalation.

Fig. 2B, which is a sectional illustration taken along section line IIB-IIB in Fig. 1A clearly shows the wedge 25, which is structured maintain a minimum distance between the subject's face (for example, the upper lip) and a portion of the oral scoop 22.

Preferably, the oral nasal sampling cannula 10 is suited to the structure of a human face by having an angle, indicated by the letter α in Fig. 2B, between at least one nasal prong 18 and oral scoop element 22. The cannula may be structured with an angle between the axis of revolution of the interior part of the oral breath collection bore 26 and the axis of revolution of the interior part of at least one nasal prong 18 (this angle is indicated by the letter β). The cannula structured with a certain angle β may allow a desirable flow of the fluid being sampled.

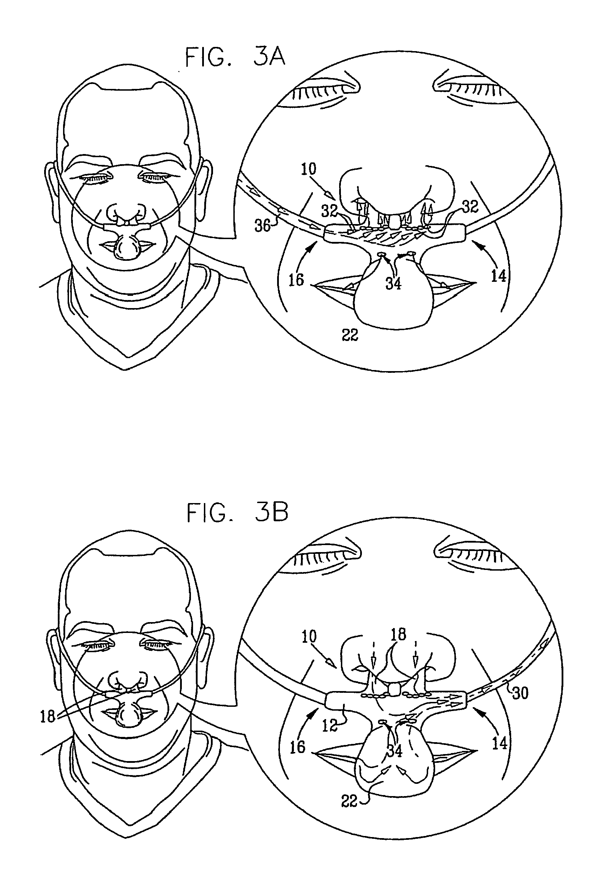

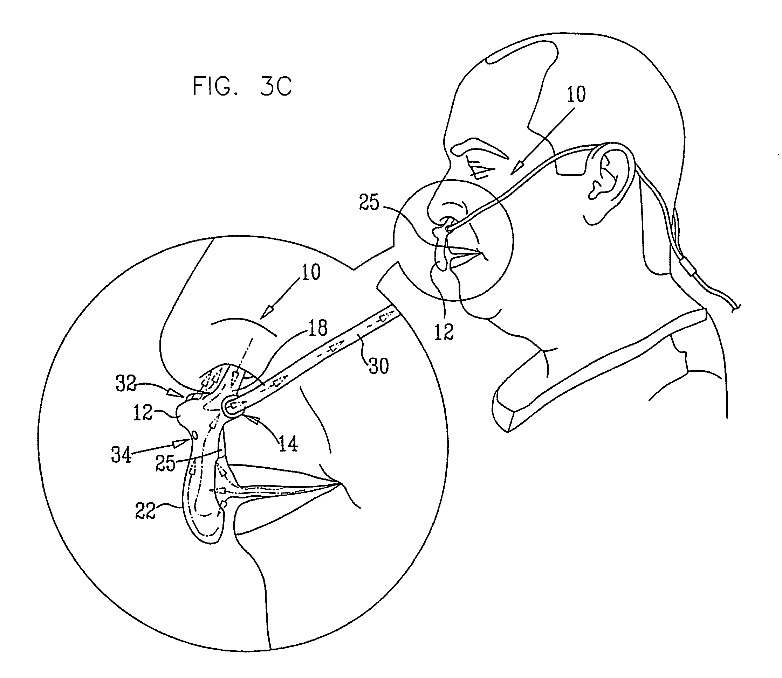

Reference is now made to Figs. 3A, 3B and 3C, which are schematic illustrations of gas flow in the oral nasal sampling cannula of Figs. 1A - 2C, wherein Fig. 3A depicts oxygen flow and Figs. 3B and 3C depict sampling of exhaled breath.

As seen in Fig. 3A, oxygen from an oxygen source (not shown) flows through oxygen delivery tube 36, through oxygen delivery bore 16 (Fig. 2B) and exits the oral nasal sampling cannula at nasal and oral oxygen delivery openings 32 and 34, toward the nose and mouth of the subject. Oral oxygen delivery openings 34 are slightly slanted, to ensure that emitted oxygen will be directed to the mouth of the subject at least partially around the oral scoop element 22.

Turning to Fig. 3B, it is seen that breath exhaled through the subject's nose is directed through nasal prongs 18 and nasal breath collection bores 21 (Fig. 2A) toward exhaled breath collection bore 14 (Fig. 2A). In a similar manner, breath exhaled through the subject's mouth is collected in oral scoop element 22, and is directed through oral breath collection bore 26 (Fig. 2B) to exhaled breath collection bore 14. All the exhaled breath collected in exhaled breath collection bore 14 flows into exhaled breath collection tube 30, typically by means of negative pressure supplied by a pumping element (not shown), which may be connected to exhaled breath collection tube 30.

Fig. 3C shows the aerodynamic nature of internal surface 24 (Fig. 1B) of oral scoop element 22. As seen in Fig. 3C, breath exhaled from the subject's mouth hits different points on the internal surface 24 of oral scoop element 22. The multiple different flow surfaces of internal surface 24 ensure that all the exhaled breath that reaches internal surface 24 will be directed toward oral breath collection bore 26 (Fig- 2B), Also shown in Fig 3C is the wedge 25 that allows increasing the gap between the oral scoop element 22 and the subject's mouth and thus prevents the suction of the oral scoop element 22 into the subject's mouth.

It is appreciated that the importance of the use of several nasal oxygen delivery openings 32 is that during exhalation, which is the period at which the subject's exhaled breath is sampled, it is crucial that the sampled breath is substantially not diluted by the oxygen that is being delivered. In the oral nasal sampling cannula 10, the positive pressure caused by the exhalation is used to push away at least most of the oxygen from the direction of the nostril, thereby ensuring that the majority of the oxygen is not sucked into the nasal prongs 18 and does not dilute the sampled breath. The use of several nasal oxygen delivery openings 32 spreads out the pressure of the oxygen flow, and thus the exhaled air is at an even larger positive pressure relative to the pressure of the oxygen exiting each delivery opening 32, thus more effectively pushing away the oxygen.

It is appreciated that the importance of the use of an oral scoop element is in the fact that a larger percentage of the orally exhaled breath is collected and eventually reaches the sample analysis element This feature is especially important when monitoring the breath of heavily sedated subjects, which tend to breathe through an open mouth and to have a very low breath rate, typically fewer than 10 breaths per minute, as opposed to greater than 12 breaths per minute in a non-sedated subject Additionally, the collection of all the exhaled breath from oral scoop element 22 into the oral breath collection bore 26, which is substantially narrower than oral scoop element 22, amplifies the pressure of the orally exhaled breath, which is typically very low, specifically in sedated subjects.

Moreover, amplification of the pressure of orally exhaled breath is important for the accuracy of the sampling due to the fact that the pressure created during exhalation at the exit of a mouth which is wide open is much lower than the pressure created by the flow of exhaled breath via the nostrils.

It is also appreciated that the sampled exhaled breath is substantially not diluted by ambient air due to pressure gradients within the system, and a majority of the sampled exhaled breath does not escape from the system.

If the subject is performing oral and nasal breathing, there may be slightly higher pressure in nasal breath collection bores 21 (Fig. 2A) and in oral breath collection bore 26 (Fig. 2B), and a slightly more negative pressure in exhaled breath collection bore 14 (Figs. 1B - 2B) due to the suctioning pump which is connected to exhaled breath collection tube 30, thereby ensuring that the exhaled breath is removed from the oral nasal sampling cannula 10 and is preferably transported towards a capnograph. Due to the relatively higher pressure within the oral scoop element 22, essentially no ambient air enters breath collection bores 21 and 26 and the exhaled breath is substantially not diluted. In the case of nasal breath only, the air in oral scoop element 22 is of the same pressure as the air all around it, whereas there is a slightly higher pressure in the nasal breath collection bores 21 pushing down via the single junction 28 (Fig. 2A), to create a relatively positive pressure at the oral breath collection bore 26, thereby ensuring that essentially no ambient air will enter the oral nasal sampling cannula 10. Additionally, essentially a majority of the exhaled breath does not escape the system due to the pumping element that constantly creates a relatively negative pressure in exhaled breath collection bore 14, thereby ensuring that essentially most of the exhaled breath will travel toward the exhaled breath collection tube 30 and not out toward the ambient air.

In a similar manner, in the case of oral breath only, the air in nasal prongs 18 and in nasal breath collection bores 21 is of the same pressure as the air all around it, whereas there is a slightly higher pressure in the oral breath collection bore 26 pushing up via the single junction 28 (Fig. 2A), to create a relatively positive pressure at the nasal breath collection bores 21, thereby ensuring that essentially no ambient air will enter the system. Additionally, essentially a majority of exhaled breath does not escape the system due to the pumping element that constantly creates a relatively negative pressure in exhaled breath collection bore 26, thereby ensuring that essentially most of the exhaled breath will travel toward the exhaled breath collection tube 30 and not out toward the ambient air.

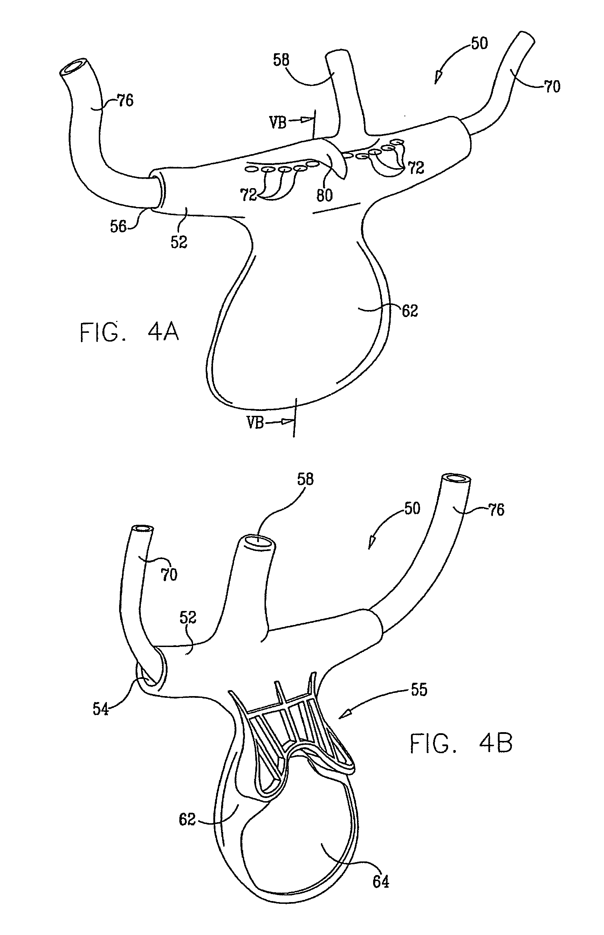

Reference is now made to Figs. 4A and 4B, which are simplified front-view and rear-view pictorial illustrations of an oral nasal sampling cannula having a single nasal prong, constructed and operative in accordance with another preferred embodiment of the present disclosure and to Figs. 5A - 5C, which are simplified sectional illustrations thereof.

Figs. 4A - 5C show an oral nasal sampling cannula 50, which is adapted for collection of gases, such as carbon dioxide, exhaled by a subject, and for supplying oxygen to the subject.

The oral nasal sampling cannula 50 comprises a main body portion 52, having formed therein an exhaled breath collection bore 54 and an oxygen delivery bore 56. A hollow nasal prong 58, having an inner end 60 which is in fluid flow communication with a nasal breath collection bore 61, is adapted for at least partial insertion into one nostril of the subject and is integrally formed with the main body portion 52.

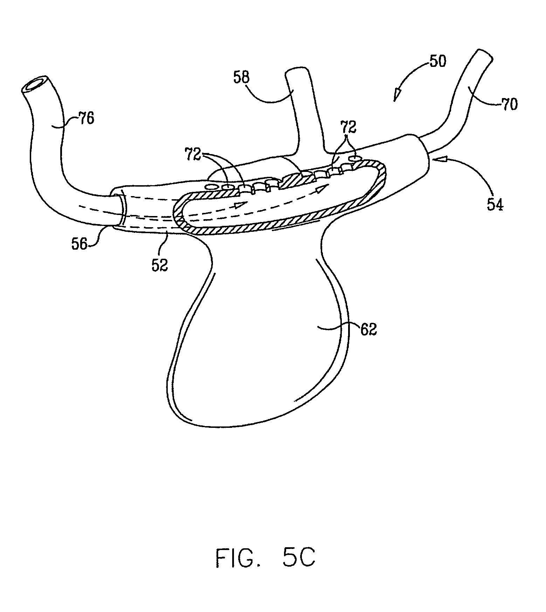

An oral scoop element 62, including an internal surface 64, which may be integrally formed with main body portion 52. Oral scoop element 62 terminates at a top portion thereof in an oral breath collection bore 66, which is in fluid flow connection with nasal breath collection bore 61, thereby forming a junction 68. The oral scoop element 62 also includes an internal surface 64, a spacer, formed in the shape of a wedge 65 adapted to maintain a minimum distance between a portion of an oral cavity and a portion of the oral scoop 62. The surface of the wedge 65 may be non-smooth, contoured and/or include structural elements such as rigids, holes, bars, nibs and the like, to form additional structural rigidity, to allow fixed seating against the face (for example, the lip), to allow moisture (for example, sweat) evaporation, to allow fixed seating against the face for subjects having facial hair, to provide comfort and/or to avoid sliding (for example, lateral sliding) of the oral nasal sampling cannula 10 on the face of the subject being examined. Fig. 4B shows examples of rigids 57 that may form spaces 59 between them. The oral scoop element 62 may be integrally formed with main body portion 52. The oral scoop element 62 terminates at a top portion thereof in an oral breath collection bore 66, which is in fluid flow connection with nasal breath collection bore 61, thereby forming an essentially single junction 68. The junction can be located above the position shown in Fig. 5A or in any other place that would allow the desired fluid flow. Fig. 5C shows a space extending from the oxygen delivery bore 66, which is in fluid flow communication with an oxygen delivery tube 70 and exits the oral nasal sampling cannula at nasal and possibly oral (not shown) oxygen delivery openings 72, toward the nose and mouth of the subject.

Junction 68 is in fluid flow communication with exhaled breath collection bore 54, which in turn is in fluid flow communication with an exhaled breath collection tube 70, which is adapted to be connected to a suctioning pump, such as that used in a side-stream capnograph (not shown), for example Microcap®, which is commercially available from Oridion BreathlD of Jerusalem, Israel.

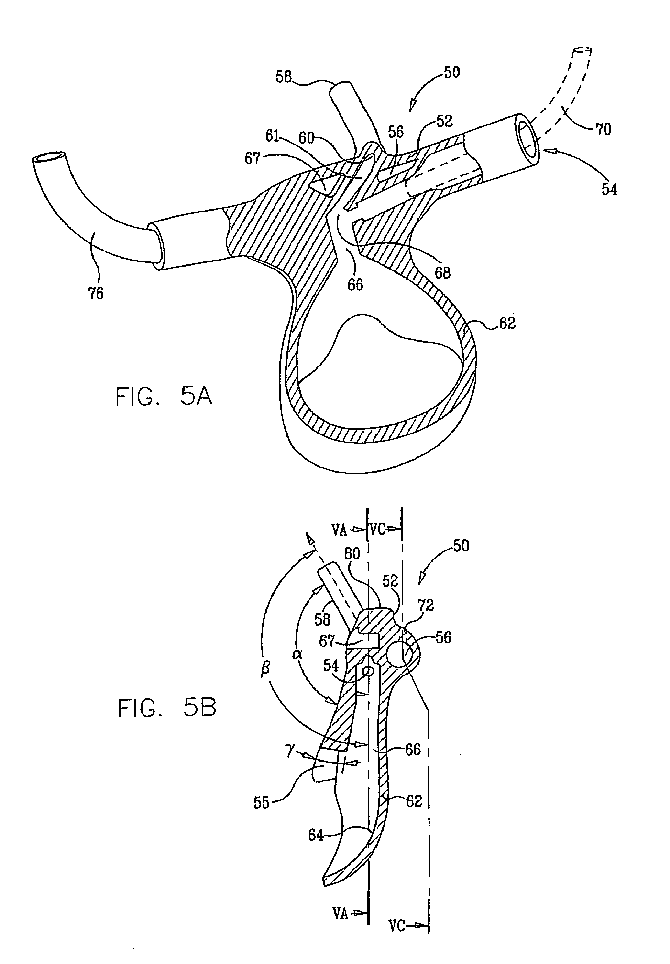

Main body portion 52, may include, preferably at a forward facing surface thereof, or alternatively at any other suitable location, nasal oxygen delivery openings 72 which are in fluid flow communication with oxygen delivery bore 56, as seen with particular clarity in Fig. 5B. Oxygen delivery bore 56, is in fluid flow communication with an oxygen delivery tube 76, which is adapted to be connected to a source of oxygen (not shown).

Oxygen delivery tube 76 and exhaled breath collection tube 70 may optionally be placed around the ears of the subject, thereby stabilizing the oral nasal sampling cannula 50 on the subject's face.

As seen clearly in Fig. 4A, a separator 80 is integrally formed with main body portion 52 at a forward facing surface thereof. Separator 80 is adapted to engage the nose of the subject, thereby distancing the nose from nasal oxygen delivery openings 72 and ensuring that a sufficient oxygen supply reaches the subject's nose, while not closing off the subject's nasal opening, which would incur a resistance to air flow during exhalation.

Fig. 5B, which is a sectional illustration taken along section line VB-VB in Fig. 4A clearly shows the wedge 55, which is structured maintain a minimum distance between the subject's face (for example, the upper lip) and a portion of the oral scoop 62. Also shown in Fig. 5B a hole 67 which may function as a structural element.

Preferably, the oral nasal sampling cannula 50 is suited to the structure of a human face by having an angle, indicated by the letter α in Fig. 5B, between the nasal prong 58 and oral scoop element 62. The cannula may be structured with an angle between the axis of revolution of the interior part of the oral breath collection bore 66 and the axis of revolution of the interior part the nasal prong 58 (this angle is indicated by the letter β). The cannula structured with a certain angle β may allow a desirable flow of the fluid being sampled. Angle β may also be defined as the angle between the diameter line of the nasal prong 58 with the center of the oral breath collection bore 66 at line VA intersect with line VB.

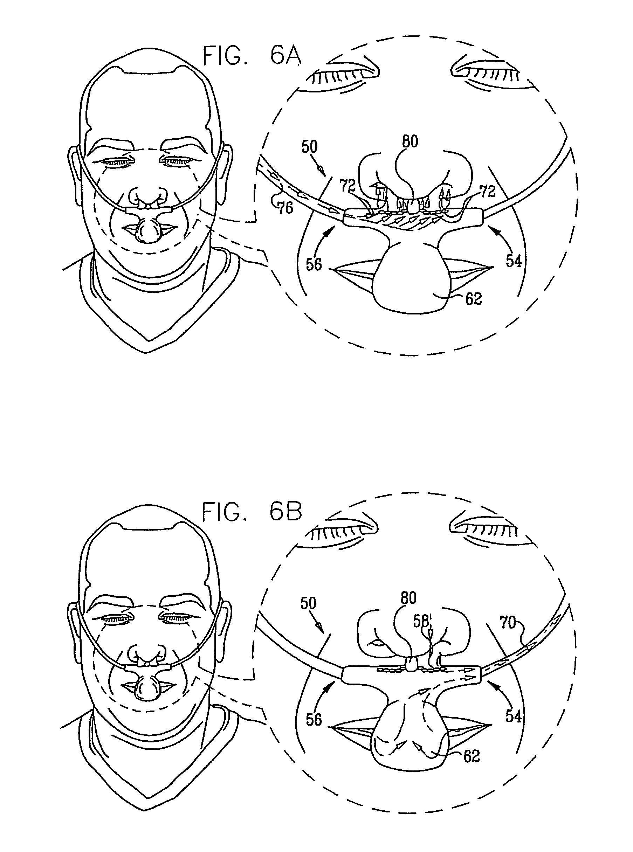

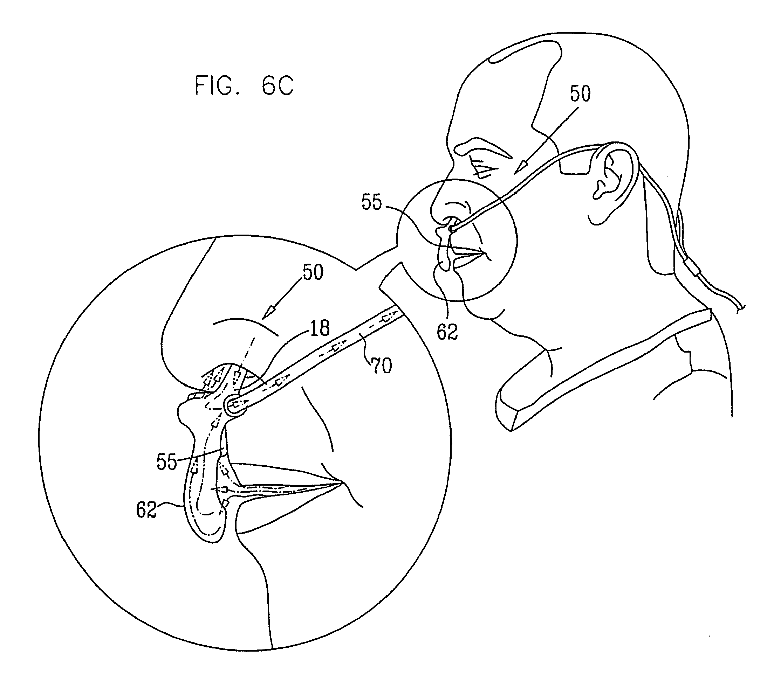

Reference is now made to Figs. 6A, 6B and 6C, which are schematic illustrations of gas flow in the oral nasal sampling cannula of Figs. 4A - 5C, wherein Fig. 6A depicts oxygen flow and Figs. 6B and 6C depict sampling of exhaled breath.

As seen in Fig. 6A, oxygen from an oxygen source (not shown) flows through oxygen delivery tube 76, through oxygen delivery bore 56 (Fig. 5B) and exits the oral nasal sampling cannula 50 at nasal oxygen delivery openings 72, toward the nose of the subject.

Turning to Fig. 6B, it is seen that breath exhaled through the subject's nose is directed through nasal prong 58 and nasal breath collection bore 61 (Fig. 5A) toward exhaled breath collection bore 54 (Fig. 5A). In a similar manner, breath exhaled through the subject's mouth is collected in oral scoop element 62, and is directed through oral breath collection bore 66 (Fig. 5B) to exhaled breath collection bore 54. All the exhaled breath collected in exhaled breath collection bore 54 flows into exhaled breath collection tube 70, typically by means of negative pressure supplied by a pumping element (not shown) which may be connected to exhaled breath collection tube 70.

Fig. 6C shows the aerodynamic nature of internal surface 64 (Fig. 4B) of oral scoop element 62. As seen in Fig. 6C, breath exhaled from the subject's mouth hits different points on the internal surface 64 of oral scoop element 62. The multiple different flow surfaces of internal surface 64 ensure that all the exhaled breath that reaches internal surface 64 will be directed toward oral breath collection bore 66 (Fig. 5B). Also shown in Fig 6C is the wedge 65 that allows increasing the gap between the oral scoop element 62 and the subject's mouth and thus prevents the suction of the oral scoop element 62 into the subject's mouth.

It is appreciated that the importance of the use of several nasal oxygen delivery openings 72 is that during exhalation, which is the period at which the subject's exhaled breath is sampled, it is crucial that the sampled breath is substantially not diluted by the oxygen that is being delivered. In the oral nasal sampling cannula 50, the positive pressure caused by the exhalation is used to push away at least most of the oxygen from the direction of the nostril, thereby ensuring that the majority of the oxygen is not sucked into the nasal prongs 58 and does not dilute the sampled breath. The use of several nasal oxygen delivery openings 72 spreads out the pressure of the oxygen flow, and thus the exhaled air is at an even larger positive pressure relative to the pressure of the oxygen exiting each delivery opening 72, thus more effectively pushing away the oxygen.

It is appreciated that the importance of the use of an oral scoop element is in the fact that a larger percentage of the orally exhaled breath is collected and eventually reaches the sample analysis element. This feature is especially important when monitoring the breath of heavily sedated subjects, which tend to breathe through an open mouth and to have a very low breath rate, typically fewer than 10 breaths per minute, as opposed to greater than 12 breaths per minute in a non-sedated subject.

Additionally, the collection of all the exhaled breath from oral scoop element 62 into the oral breath collection bore 66, which is substantially narrower than oral scoop element 62, amplifies the pressure of the orally exhaled breath, which is typically very low, specifically in sedated subjects.

Moreover, amplification of the pressure of orally exhaled breath is important for the accuracy of the sampling due to the fact that the pressure created during exhalation at the exit of a mouth which is wide open is much lower than the pressure created by the flow of exhaled breath via the nostril.

It is also appreciated that the sampled exhaled breath is substantially not diluted by ambient air due to pressure gradients within the system, and a majority of the sampled exhaled breath does not escape from the system.

If the subject is performing oral and nasal breathing, there is a slightly higher pressure in nasal breath collection bore 61 (Fig. 5A) and in oral breath collection bore 66 (Fig. 5B), and a slightly more negative pressure in exhaled breath collection bore 54 (Fig. 5A) due to the suctioning pump which is connected to exhaled breath collection tube 70, thereby ensuring that the exhaled breath is removed from the oral nasal sampling cannula 50 and is preferably transported towards a capnograph. Due to positive pressure within the oral scoop element, essentially no ambient air enters breath collection bores 61 and 66 and the exhaled breath is essentially not diluted.

In the case of nasal breath only, the air in oral scoop element 62 is of the same pressure as the air all around it, whereas there is slightly higher pressure in the nasal breath collection bore 61 pushing down via the junction 68 (Fig, 5A), to create a relatively positive pressure at the oral breath collection bore 66, thereby ensuring that essentially no ambient air will enter the system. Additionally, essentially most of the exhaled breath does not escape the system due to the pumping element that constantly creates a relatively low pressure in exhaled breath collection bore, thereby ensuring that essentially most of the exhaled breath will travel toward the exhaled breath collection tube 70 and not out toward the ambient air.

In a similar manner, in the case of oral breath only, the air in nasal prong 58 and in nasal breath collection bore 61 is of the same pressure as the air all around it, whereas there is a slightly higher pressure in the oral breath collection bore 66 pushing up via the junction 68, to create a relatively positive pressure at the nasal breath collection bore 61, thereby ensuring that essentially no ambient air will enter the system. Additionally, essentially a majority of the exhaled breath does not escape the system due to the pumping element that constantly creates a relatively negative pressure in exhaled breath collection bore, thereby ensuring that essentially most of the exhaled breath will travel toward the exhaled breath collection tube 70 and not out toward the ambient air.

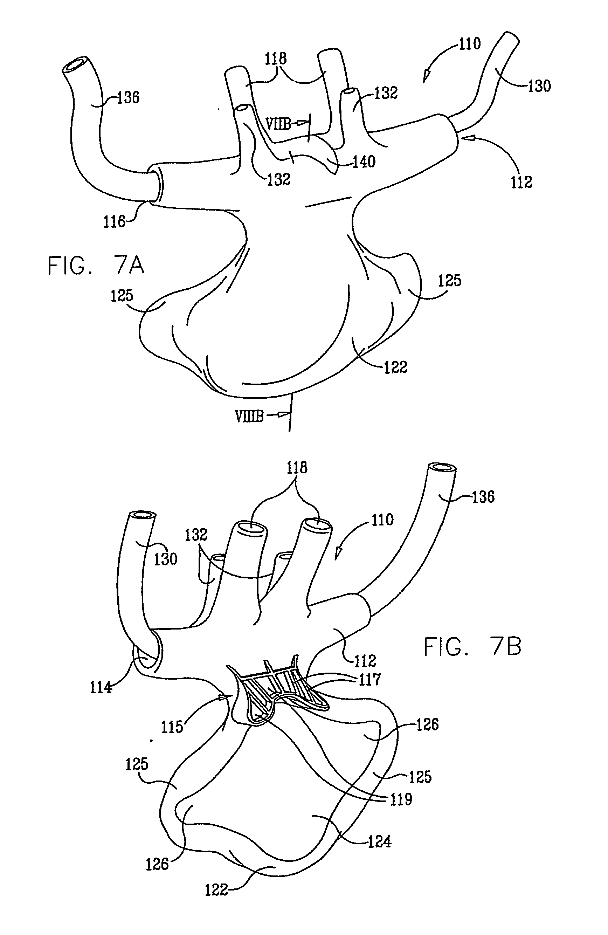

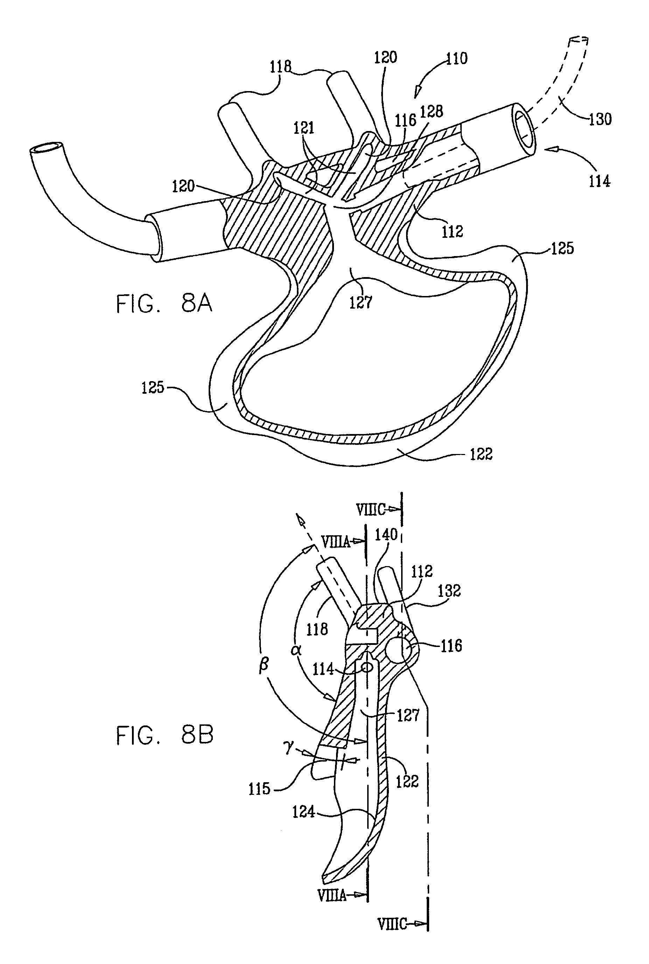

Reference is now made to Figs. 7A and 7B, which are simplified front-view and rear-view pictorial illustrations of an oral nasal sampling cannula having an enlarged oral scoop, which is constructed and operative in accordance with a preferred embodiment of the present disclosure and to Figs. 8A and 8B, which are simplified sectional illustrations thereof.

Figs. 7A - 8C show an oral nasal sampling cannula 110, which is adapted for collection of gases, such as carbon dioxide, exhaled by a subject, and for supplying oxygen to the subject.

The oral nasal sampling cannula 110 comprises a main body portion 112, having formed therein an exhaled breath collection bore 114 and an oxygen delivery bore 116. A pair of hollow nasal prongs 118, having inner ends 120, which are in fluid flow communication with a pair of nasal breath collection bores 121, is adapted for at least partial insertion into the nostrils of the subject and is integrally formed with the main body portion 112.

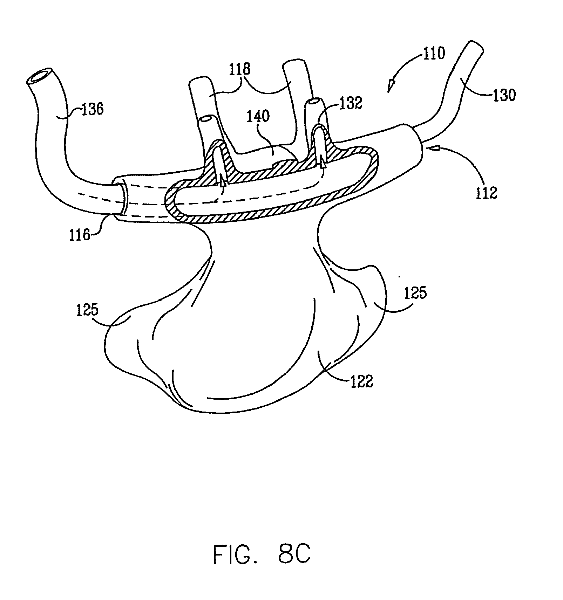

An oral scoop element 122, including an internal surface 124, is integrally formed with main body portion 112. Oral scoop element 122 additionally has formed thereon a pair of extension portions 125, each having an internal surface 126, and terminates at a top portion thereof in an oral breath collection bore 127. Oral breath collection bore 127 is in fluid flow connection with nasal breath collection bores 121, thereby forming a single junction 128. The oral scoop element 122 also includes an internal surface 124, a spacer, formed in the shape of a wedge 115 adapted to maintain a minimum distance between a portion of an oral cavity and a portion of the oral scoop 122. The surface of the wedge 115 may be non-smooth, contoured and/or include structural elements such as rigids, holes, bars, nibs and the like, to form additional structural rigidity, to allow fixed seating against the face (for example, the lip), to allow moisture (for example, sweat) evaporation, to allow fixed seating against the face for subjects having facial hair, to provide comfort and/or to avoid sliding (for example, lateral sliding) of the oral nasal sampling cannula 10 on the face of the subject being examined. Fig. 7B shows examples of rigids 117 that may form spaces 119 between them. The oral scoop element 122 may be integrally formed with main body portion 112. The oral scoop element 122 terminates at a top portion thereof in an oral breath collection bore 127, which is in fluid flow connection with nasal breath collection bores 121, thereby forming an essentially single junction 128. The junction can be located above the position shown in Figs. 8A or in any other place that would allow the desired fluid flow. Fig. 8C shows the space 913 extending from the oxygen delivery bore 127, which is in fluid flow communication with an oxygen delivery tube 130 and exits the oral nasal sampling cannula 110 at nasal oxygen delivery prongs 132, toward the nose of the subject.

Single junction 128 is in fluid flow communication with exhaled breath collection bore 114, which in turn is in fluid flow communication with an exhaled breath collection tube 130, which is adapted to be connected to a suctioning pump, such as that used in a side-stream capnograph (not shown), for example Microcap®, which is commercially available from Oridion BreathlD of Jerusalem, Israel.

Main body portion 112 includes, preferably at a forward facing surface thereof or alternatively at any other suitable location, nasal oxygen delivery prongs 132 which are typically shorter than nasal prongs 118 such that they do not enter the subject's nostrils. The exits of the nasal oxygen delivery prongs 132 facing the nostrils may have different shapes, for example a funnel shape. The nasal oxygen delivery prongs 132 are in fluid flow communication with oxygen delivery bore 116, as seen with particular clarity in Fig. 8B. Oxygen delivery bore 116 is in fluid flow communication with an oxygen delivery tube 136, which is adapted to be connected to a source of oxygen (not shown).

Oxygen delivery tube 136 and exhaled breath collection tube 130 may optionally be placed around the ears of the subject, thereby stabilizing the oral nasal sampling cannula 110 on the subject's face.

As seen clearly in Fig. 7A, a separator 140 is integrally formed with main body portion 112 at a forward facing surface thereof. Separator 140 is adapted to engage the nose of the subject, thereby distancing the nostrils from nasal oxygen delivery prongs 132 and ensuring that a sufficient oxygen supply reaches the subject's nose, while not closing off the subject's nasal opening, which would incur a resistance to air flow during exhalation.

Fig. 8B, which is a sectional illustration taken along section line VIIB-VIIB in Fig. 7A clearly shows the wedge 115, which is structured maintain a minimum distance between the subject's face (for example, the upper lip) and a portion of the oral scoop 122. Also shown in Fig. 5B a hole 117 which may function as a structural element.

Preferably, the oral nasal sampling cannula 110 is suited to the structure of a human face by having an angle, indicated by the letter α in Fig. 8B, between the at least one nasal prong 118 and oral scoop element 122. The cannula may be structured with an angle between the axis of revolution of the interior part of the oral breath collection bore 127 and the axis of revolution of the interior part of at least one of the nasal prong 118 (this angle is indicated by the letter β). The cannula structured with a certain angle β may allow a desirable flow of the fluid being sampled. Angle β may also be defined as the angle between the diameter line of the nasal prong 118 with the center of the oral breath collection bore 127 at line VIIA intersect with line VIIB.

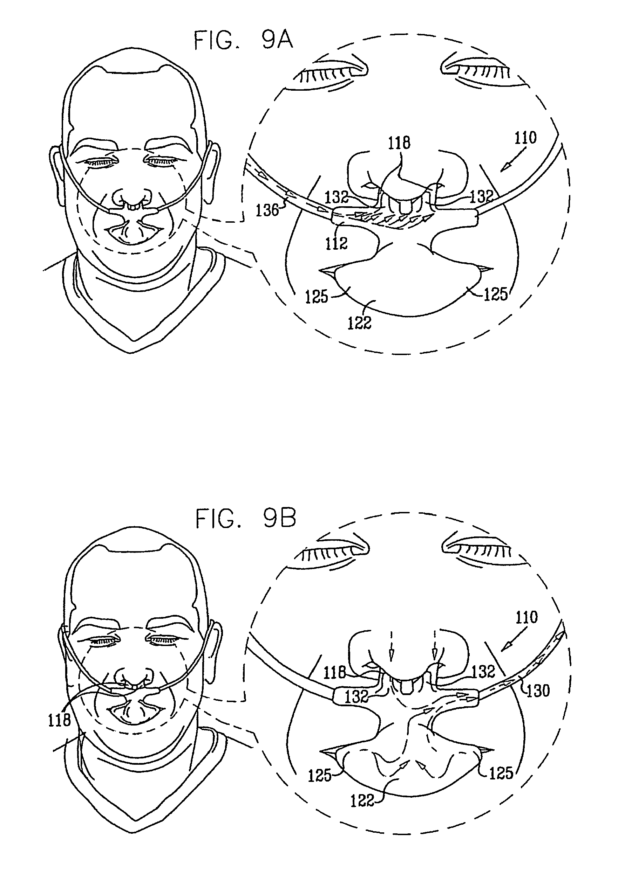

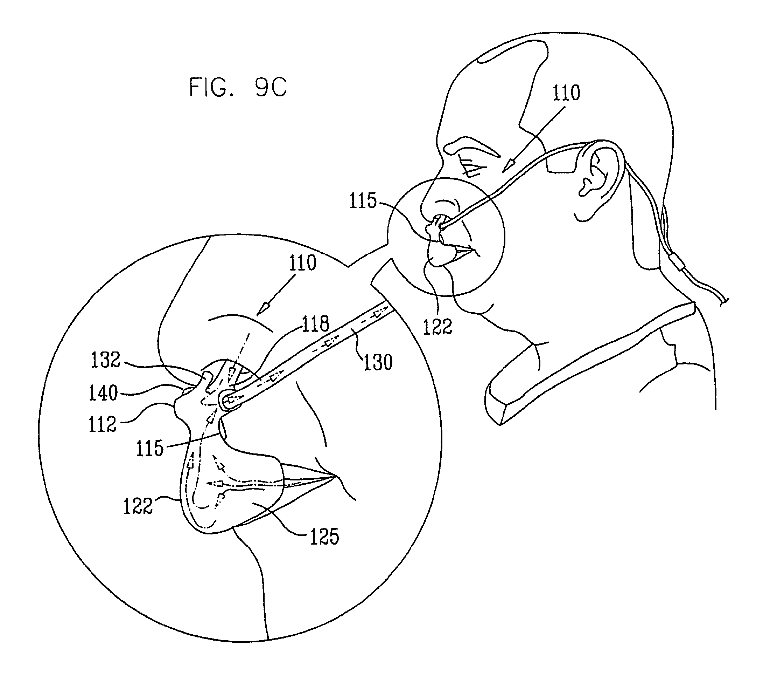

Reference is now made to Figs. 9A, 9B and 9C, which are schematic illustrations of gas flow in the oral nasal sampling cannula 110 of Figs. 7A - 8C, wherein Fig. 9A depicts oxygen flow and Figs. 9B and 9C depict sampling of exhaled breath.

As seen in Fig. 9A, oxygen from an oxygen source (not shown) flows through oxygen delivery tube 136, through oxygen delivery bore 116 (Fig. 8B) and exits the oral nasal sampling cannula 110 at nasal oxygen delivery prongs 132, toward the nose of the subject.

Turning to Fig. 9B, it is seen that breath exhaled through the subject's nose is directed through nasal prongs 118 and nasal breath collection bores 121 (Fig. 8A) toward exhaled breath collection bore 114 (Fig. 8A). In a similar manner, breath exhaled through the subject's mouth is collected by oral scoop element 122 and by extension portions 125, and is directed through oral breath collection bore 127 (Fig. 8B) to exhaled breath collection bore 114. All of the exhaled breath collected in exhaled breath collection bore 114 flows into exhaled breath collection tube 130, typically by means of negative pressure supplied by a pumping element (not shown) which may be connected to exhaled breath collection tube 130.

Fig. 9C shows the aerodynamic nature of internal surfaces 124 and 126 (Figs. 7B) of oral scoop element 122 and extension portions 125 thereof. As seen in Fig. 9C, breath exhaled from the subject's mouth hits different points on the internal surfaces 124 and 126 of oral scoop element 122 and extension portions 125 thereof. The multiple different flow surfaces of internal surfaces 124 and 126 ensure that all the exhaled breath that reaches internal surfaces 124 and 126 will be directed toward oral breath collection bore 127 (Fig. 8B).

It is appreciated that the nasal oxygen delivery prongs 132 are shorter than the nasal prongs 118 such that during exhalation, which is the period at which the subject's exhaled breath is sampled, it is crucial that the sampled breath is substantially not diluted by the oxygen that is being delivered. In the oral nasal sampling cannula 110, the positive pressure caused by the exhalation is used to push away at least a majority of the oxygen from the direction of the nostril, thereby ensuring that most of the delivered oxygen is not sucked into the nasal prongs 118 and essentially does not dilute the sampled breath. If the nasal oxygen delivery prongs 132 were at the same height as the nasal prongs 118, even if the oxygen were pushed back and away during exhalation, some oxygen would still enter the sampling nasal prongs 118 thereby diluting the sample. The fact that the nasal oxygen delivery prongs 132 are lower than sampling nasal prongs 118 prevents this from occurring.

It is appreciated that the importance of the use of an oral scoop element is in the fact that a larger percentage of the orally exhaled breath is collected and eventually reaches the sample analysis element. The use of extension portions 125 ensures that generally an oral breath collection device covers a majority of the subject's mouth, thereby collecting most of the subject's orally exhaled breath. These features are especially important when monitoring the breath of heavily sedated subjects, which tend to breathe through an open mouth and to have a very low breath rate, typically fewer than 10 breaths per minute, as opposed to greater than 12 breaths per minute in a non-sedated subject.

Additionally, the collection of most of the exhaled breath from oral scoop element 122 and extension portions 125 into the oral breath collection bore 127, which is substantially narrower than oral scoop element 122 and extension portions 125 thereof, amplifies the pressure of the orally exhaled breath, which is typically very low, specifically in sedated subjects.

Moreover, amplification of the pressure of orally exhaled breath is important for the accuracy of the sampling due to the fact that the pressure created during exhalation at the exit of a mouth which is wide open is much lower than the pressure created by the flow of exhaled breath via the nostrils.

It is also appreciated that the sampled exhaled breath is substantially not diluted by ambient air due to pressure gradients within the system, and a majority of the sampled exhaled breath does not escape from the system.

If the subject is performing oral and nasal breathing, there is slightly higher pressure in nasal breath collection bores 121 (Fig. 8A) and in oral breath collection bore 127 (Fig. 8B), and slightly more negative pressure in exhaled breath collection bore 114 (Fig. 8A) due to the suctioning pump which is connected to exhaled breath collection tube 130, thereby ensuring that at least most of the exhaled breath is removed from the oral nasal sampling cannula 110 and is preferably transported towards a gas analyzer such as a capnograph. Due to the relatively positive pressure within the oral scoop element 122, essentially no ambient air enters breath collection bores 121 and 127 and the exhaled breath is substantially not diluted.

In the case of nasal breath only, the air in oral scoop element 122 and in extension portions 125 is of the same pressure as the air all around it, whereas there is slightly higher pressure in the nasal breath collection bores 121, thereby ensuring that essentially no ambient air will enter the oral nasal sampling cannula 110. Additionally, essentially a majority of the exhaled breath does not escape the system due to the pumping element that constantly creates a relatively negative pressure in exhaled breath collection bore, thereby ensuring that most of the exhaled breath will travel toward the exhaled breath collection tube 130 and not out toward the ambient air.

In a similar manner, in the case of oral breath only, the air in nasal prongs 118 and in nasal breath collection bores 121 is of the same pressure as the air all around it, whereas there is slightly higher pressure in the oral breath collection bore 127 pushing up via the single junction 128, to create a relatively positive pressure at the nasal breath collection bores 121, thereby ensuring that essentially no ambient air will enter the oral nasal sampling cannula 110. Additionally, essentially a majority of the exhaled breath does not escape the system due to the pumping element that constantly creates a relatively negative pressure in exhaled breath collection bore, thereby ensuring that most of the exhaled breath will travel toward the exhaled breath collection tube 130 and not out toward the ambient air.

It is appreciated by persons skilled in the art that the present disclosure is not limited by what has been particularly shown and described hereinabove. Rather the scope of the present disclosure includes both combinations and subcombinations of various features described hereinabove as well as variations and modifications thereto which would occur to a person of skill in the art upon reading the above description and which are not in the prior art.

In addition to the exemplary aspects and embodiments described above, further aspects and embodiments will become apparent by reference to the figures and by study of the following detailed descriptions.

## Claims

1. A cannula comprising:
a spacer adapted to maintain a minimum distance between at least a portion of an oral cavity and at least a portion of an oral breath collector.

2. The cannula of claim 1, further comprising at least one nasal breath collector.

3. The cannula of claim 1, wherein said spacer is shaped in a form of a wedge.

4. The cannula of claim 2, wherein said wedge comprises an upper edge and a lower edge, wherein the upper edge is thinner than the lower thick edge.

5. The cannula of claim 1, wherein said at least a portion of oral cavity comprises an upper lip.

6. The cannula of claim 1, wherein said oral breath collector comprises a scoop adapted to collect orally exhaled breath.

7. The cannula of claim 1, wherein said oral breath collector is adapted to direct oral exhaled toward a suction port.

8. The cannula of claim 1, wherein said oral breath collector is adapted to cover a substantial portion of the oral cavity.

9. The cannula of claim 2, wherein said nasal breath collector comprises a nasal prong which is adapted to be at least partially inserted into a nostril.

10. The cannula of claim 1, further comprising at least one oral oxygen delivery port.

11. The cannula of claim 10, wherein said at least one oral oxygen delivery port is adapted to deliver oxygen around said oral scoop.

12. The cannula of claim 1, further comprising at least one nasal oxygen delivery port.

13. The cannula of claim 12, wherein said at least one nasal oxygen delivery port comprises a plurality of oxygen delivery holes.

14. The cannula of claim 12, wherein said at least one nasal oxygen delivery port comprises a nasal oxygen delivery prong adapted to be at least partially inserted into a nostril of a subject.

15. The cannula of claim 12, further comprising a separator, adapted to maintain a predetermined distance between said at least one nasal oxygen delivery port and a nostril of a subject.

16. The cannula of claim 2, being formed with an angle between said at least one oral breath collector and said nasal breath collector.

17. The cannula of claim 16, wherein said angle is in the range of 125 - 145 degrees.

18. A cannula comprising a spacer adapted to maintain a minimum distance between at least a portion of an oral cavity and at least a portion of an oral breath collector, wherein said cannula is coupled to a breath test analyzer.

19. The cannula of claim 18, further comprising at least one nasal breath collector.

20. A method for sampling exhaled breath comprising:
positioning a cannula on a face of a subject, wherein the cannula comprises a spacer adapted to maintain a minimum distance between at least a portion of an oral cavity and at least a portion of an oral breath collector.

21. The method of claim 20, further comprising at least one nasal breath collector.

22. The method of claim 20, wherein said spacer is shaped in a form of a wedge.

23. The method of claim 22, wherein said wedge comprises an upper thin edge and a lower thick edge.

24. The method of claim 22, wherein said at least a portion of oral cavity comprises an upper lip.

25. The method of claim 21, wherein said positioning comprising forming an angle between said at least one oral breath collector and said nasal breath collector.

26. The method of claim 25, wherein said angle is in the range of 125 - 145 degrees.

27. The method of claim 21, further comprising at least partially inserting said at least one nasal breath collector into a nostril.

28. The method of claim 27, further comprising sampling exhaled breath collected from said oral cavity, said nostril or both using a gas analyzer coupled to said cannula.

29. The method of claim 28, further comprising providing oxygen through a nasal oxygen delivery port, an oral oxygen delivery port or both.
